# EUROPEAN PATENT SPECIFICATION

(11) **EP 3 849 550 B1**
(45) Date of publication and mention of the grant of the patent: **07.08.2024**
(21) Application number: 19861171.7
(22) Date of filing: 13.09.2019
(51) Int. Cl.: A61K 31/437, A61K 31/506, A61K 31/519, A61K 31/565, A61P 35/00, C07D 401/14, C07D 471/04, C07D 487/04, C07J 31/00, A61K 45/06

(54) **COMBINATION THERAPY FOR THE TREATMENT OF ESTROGEN-RECEPTOR POSITIVE BREAST CANCER**
KOMBINATIONSTHERAPIE ZUR BEHANDLUNG VON ÖSTROGEN-REZEPTOR-POSITIVEM BRUSTKREBS
POLYTHÉRAPIE POUR LE TRAITEMENT DU CANCER DU SEIN POSITIF AU RÉCEPTEUR DES OESTROGÈNES

(30) Priority: 13.09.2018 US 201862730837 P
(43) Date of publication of application: 21.07.2021
(73) Proprietor: Zenith Epigenetics Ltd., Calgary, AB T3E 6L1 (CA)
(72) Inventor: CAMPEAU, Eric, Calgary, Alberta T3A 1S4 (CA); KHARENKO, Olesya, Calgary, Alberta T2Z 1G6 (CA); VAN DER HORST, Edward, T.H., Palo Alto, CA 94304 (US)
(74) Representative: Mathys & Squire
(86) International application number: PCT/IB2019/001035
(87) International publication number: WO 2020/053664

(56) References cited:
- WO-A1-2018/097977
- WO-A1-2018/106444
- WO-A2-2015/002754
- WO-A2-2015/002754
- WO-A2-2015/074064
- KHARENKO OLESYA ET AL: "Abstract P3-06-07: The BET bromodomain inhibitors ZEN-3694 and ZEN-3309 targets several mechanisms of resistance to endocrine therapies in preclinical models of ER+ breast cancers", POSTER SESSION ABSTRACTS, 14 February 2018 (2018-02-14), pages P3-06-07 - P3-06-07, XP055914119, Retrieved from the Internet <URL:https://aacrjournals.org/cancerres/article/78/4_Supplement/P3-06-07/632403/Abstract-P3-06-07-The-BET-bromodomain-inhibitors> DOI: 10.1158/1538-7445.SABCS17-P3-06-07
- ATTWELL S. ET AL: "The clinical candidate ZEN-3694, a BET bromodomain inhibitor, is efficacious in the treatment of a variety of solid tumor and hematological malignancies, alone or in combination with several standard of care therapies", 2015-AACR-EORTC, 1 January 2015 (2015-01-01), pages 1 - 1, XP055913820, Retrieved from the Internet <URL:chrome-extension://efaidnbmnnnibpcajpcglclefindmkaj/viewer.html?pdfurl=https%3A%2F%2Fwww.zenithepigenetics.com%2Fupload%2Fmedia_element%2F36%2F01%2F2015-aacr-eortc-poster.pdf&clen=1543954&chunk=true> [retrieved on 20220420]
- SAMMONS SARAH ET AL: "Fulvestrant-Based Combination Therapy for Second-Line Treatment of Hormone Receptor-Positive Advanced Breast Cancer", TARGETED ONCOLOGY, SPRINGER INTERNATIONAL PUBLISHING, CHAM, vol. 14, no. 1, 22 August 2018 (2018-08-22), pages 1 - 12, XP036750995, ISSN: 1776-2596, [retrieved on 20180822], DOI: 10.1007/S11523-018-0587-9
- KWAPISZ DOROTA: "Cyclin-dependent kinase 4/6 inhibitors in breast cancer: palbociclib, ribociclib, and abemaciclib", BREAST CANCER RESEARCH AND TREATMENT, SPRINGER US, NEW YORK, vol. 166, no. 1, 24 July 2017 (2017-07-24), pages 41 - 54, XP036343860, ISSN: 0167-6806, [retrieved on 20170724], DOI: 10.1007/S10549-017-4385-3
- ATTWELL S ET AL: "The investigational drug ZEN-3694, a novel BET-bromodomain inhibitor, inhibits multiple tumor immune escape mechanisms and has the potential to combine with immunotherapies", EUROPEAN JOURNAL OF CANCER, vol. 69, 29 November 2016 (2016-11-29), XP029843723, ISSN: 0959-8049, DOI: 10.1016/S0959-8049(16)32858-1
- COSÍN MARC ET AL: "Abstract 1282: Overcoming resistance to inhibitors of CDK4/6 and BET in estrogen receptor-positive breast cancer", MOLECULAR AND CELLULAR BIOLOGY / GENETICS, 13 August 2020 (2020-08-13), pages 1 - 1, XP055914108, Retrieved from the Internet <URL:https://aacrjournals.org/cancerres/article/80/16_Supplement/1282/641235/Abstract-1282-Overcoming-resistance-to-inhibitors> DOI: 10.1158/1538-7445.AM2020-1282
- KHARENKO OLESYA A. ET AL: "Combination of ZEN-3694 with CDK4/6 inhibitors reverses acquired resistance to CDK4/6 inhibitors in ER-positive breast cancer", CANCER GENE THERAPY, 12 August 2021 (2021-08-12), New York, XP055913799, ISSN: 0929-1903, Retrieved from the Internet <URL:https://www.nature.com/articles/s41417-021-00375-9> DOI: 10.1038/s41417-021-00375-9

## Description

The invention relates to the treatment of breast cancer and is as defined in the claims.

### BACKGROUND

More than 200,000 women are diagnosed with breast cancer every year in the United States. About 80% of these cases are estrogen receptor positive (ER+), which is characterized by the up-regulation of ER signaling. Current lines of therapies include endocrine therapies which have resulted in great treatment improvements in ER+ breast cancer. Unfortunately, resistance to these therapies occurs over time and development of additional therapeutic strategies is needed. Recently, the bromodomain and extra terminal (BET) proteins BRD3 and BRD4 were shown to be involved in the transcription of ER (Feng et al., 2014). Treatment with some BET inhibitor can suppress ER-mediated signaling, offering a potential strategy to overcome endocrine resistance by further ER-signaling suppression regardless of ESR1 mutation status (Feng et al., 2014; Ladd et al., 2016; Nagarajan et al., 2014; Sengupta et al., 2015). However, it remains unclear if BRD3 and/or BRD4 are involved in the resistance mechanisms to endocrine therapies in patients, and whether BET inhibitor can potently inhibit the proliferation of ER+ breast cancer cells that are resistant to CDK4/6 inhibitors. CDK4/6 inhibitors are standard of care in first and second line metastatic ER+ breast cancer, and a combination of a CDK4/6 inhibitor and a BET inhibitor can be next line of therapy for subjects developing resistance to the CDK4/6 mono therapy.

However, at this time, it is unclear which, if any, BET inhibitors will result in clinical benefit when administered to subjects with ER+ breast cancer. It is also unclear which, if any BET inhibitors will combine synergistically with other drugs, such as an a selective-estrogen receptor degrader (SERD), a selective-estrogen receptor modulator (SERM), an aromatase inhibitor (Al), or a selective CDK4/6 inhibitor, in the treatment of breast cancer; what level of synergy is required; and which second therapeutic agent will be the best combination partner for each BET inhibitor, resulting in clinical benefit when administered to patients with breast cancer. In addition to a clinical benefit, the combination also has to be safe and well tolerated at the efficacious doses. At this time, it cannot be predicted which combination will show the best overall profile.
Kharenko et al (Cancer Res (2018) 78 (4_Supplement): Abstract P3-06-07) reports that the BET bromodomain inhibitor ZEN-3694 targets several mechanisms of resistance to endocrine therapies in preclinical models of ER+ breast cancers. Attwell et al (2015-aacr-eortc, 1 January 2015 (2015-01-01), pages 1-1) reports on the efficacy of ZEN-3694 in the treatment of a variety of solid tumor and hematological malignancies, alone or in combination with other therapies. Sammons et al (Targeted Oncology (2019) 14:1-12) relates to fulvestrant-based combination therapy for second-line treatment of hormone receptor-positive advanced breast cancer.

### SUMMARY OF THE INVENTION

The invention is defined in the claims.
In particular, the present invention provides a BET bromodomain inhibitor selected from 1-benzyl-6-(3,5-dimethylisoxazol-4-yl)-N-methyl-1H-imidazo[4,5-b]pyridin-2-amine (Compound I) and pharmaceutically acceptable salts/co-crystals thereof, for use in a method of treating estrogen receptor positive (ER+) breast cancer in a patient, the method comprising administering said BET bromodomain inhibitor in combination with a second therapeutic agent, wherein the second therapeutic agent is fulvestrant or the second therapeutic agent is abemaciclib.

### DETAILED DESCRIPTION

The technical information set out below may in some respects go beyond the scope of the invention, which is defined by the appended claims. The additional technical information is provided to place the actual invention in a broader technical context and to illustrate possible related technical developments.
Any incidental references to methods for treatment of the human or animal body by surgery or therapy and diagnostic methods practised on the human or animal body are not to be construed as claiming protection for such methods as such, but are instead to be construed as referring to products, in particular substances or compositions, for use in any of these methods.

The present invention relates to treating estrogen receptor positive (ER+) breast cancer by concomitant administration of a BET bromodomain inhibitor, or a pharmaceutically acceptable salt or co-crystal of a BET bromodomain inhibitor, and a second therapeutic agent to a subject in need thereof. In the present invention, the BET bromodomain inhibitor is 1-benzyl-6-(3,5-dimethylisoxazol-4-yl)-N-methyl-1H-imidazo[4,5-b]pyridin-2-amine (Compound I) and the second therapeutic agent is fulvestrant or abemaciclib.

In some embodiments of the invention, the method of treating ER+ breast cancer is a triple combination therapy comprising administration of a BET bromodomain inhibitor, a second therapeutic agent, and an estrogen receptor modulator.

In some embodiments, the BET bromodomain inhibitor is administered simultaneously with the second therapeutic agent and optionally with the estrogen receptor modulator. In some embodiments, the BET bromodomain inhibitor is administered sequentially with the second therapeutic agent and optionally with the estrogen receptor modulator. In some embodiments, the BET bromodomain inhibitor is administered in a single pharmaceutical composition with the second therapeutic agent and optionally with the estrogen receptor modulator. In some embodiments, the BET bromodomain inhibitor and the second therapeutic agent and optionally the estrogen receptor modulator are administered as separate compositions. In some embodiments, the BET bromodomain inhibitor and the second therapeutic agent are in a single composition and the optional estrogen receptor modulator is in a separate composition.

As described generally herein, a BET bromodomain inhibitor may be a compound of Formula la or Formula lb 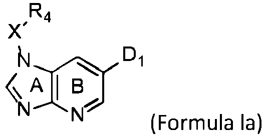 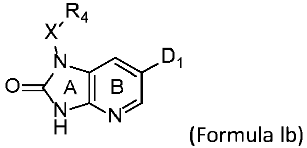 or a stereoisomer, tautomer, pharmaceutically acceptable salt, or co-crystal thereof, wherein:
**Ring A** and **Ring B** may be optionally substituted with groups independently selected from hydrogen, deuterium, -NH₂, amino, heterocycle(C₄-C₆), carbocycle(C₄-C₆), halogen, -CN, - OH, -CF₃, alkyl (C₁-C₆), thioalkyl (C₁-C₆), alkenyl (C₁-C₆), and alkoxy (C₁-C₆);
**X** is selected from -NH-, -CH₂-, -CH₂CH₂-, -CH₂CH₂CH₂-, -CH₂CH₂O-, -CH₂CH₂NH-, - CH₂CH₂S-,
   -C(O)-, -C(O)CH₂-, -C(O)CH₂CH₂-, -CH₂C(O)-, -CH₂CH₂C(O)-, -C(O)NH-, -C(O)O-, -C(O)S-, - C(O)NHCH₂-,
   -C(O)OCH₂-, -C(O)SCH₂-, wherein one or more hydrogen may independently be replaced with deuterium, hydroxyl, methyl, halogen, -CF₃, ketone, and where S may be oxidized to sulfoxide or sulfone;
**R₄** is selected from optionally substituted 3-7 membered carbocycles and heterocycles; and
**D₁** is selected from the following 5-membered monocyclic heterocycles: 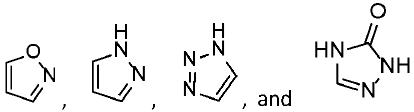 which are optionally substituted with hydrogen, deuterium, alkyl (C₁-C₄), alkoxy (C₁-C₄), amino, halogen, amide, -CF₃, -CN, -N₃, ketone (C₁-C₄), -S(O)Alkyl(C₁-C₄), -SO₂alkyl(C₁-C₄), -thioalkyl(C₁-C₄), -COOH, and/or ester, each of which may be optionally substituted with hydrogen, F, Cl, Br, -OH, -NH₂, -NHMe, -OMe, -SMe, oxo, and/or thio-oxo.

In the present invention, the BET bromodomain inhibitor is as defined in the claims. Accordingly, in some embodiments, the BET bromodomain inhibitor is a compound of Formula la. which is 1-benzyl-6-(3,5-dimethylisoxazol-4-yl)-N-methyl-1H-imidazo[4,5-b]pyridine-2-amine (Compound I), which has the following formula:

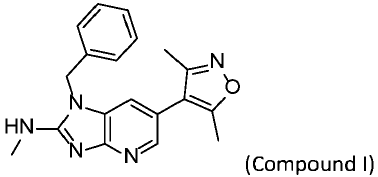

In some embodiments, the BET bromodomain inhibitor is a pharmaceutically acceptable salt or co-crystal of Compound I. In some embodiments, the BET bromodomain inhibitor is a mesylate salt/co-crystal of Compound I Form I.

### BRIEF DESCRIPTION OF THE DRAWINGS

**FIG. 1** Synergetic combination of the Compound I with abemaciclib in WT-MCF7 cells. CI=0.08
**FIG. 2** Synergetic combination of the Compound I with abemaciclib in Palbo-R-MCF7 cells. CI=0.20
**FIG. 3** Synergetic combination of the Compound I with abemaciclib in ZR-75-1 cells. CI=0.14
**FIG. 4** Synergetic combination of the Compound I with abemaciclib in Palbo-R ZR-75-1 cells. CI=0.35
**FIG. 5** Synergetic combination of the Compound I with abemaciclib in Abema-R MCF7 cells. CI=0.30
**FIG. 6** Synergetic combination of the Compound I with fulvestrant in WT-MCF7 cells. CI=0.51.
**FIG. 7** shows an X-ray powder diffractogram (XRPD) of a mesylate salt/co-crystal of Compound I.
**FIG. 8** shows a differential scanning calorimeter (DSC) curve of a mesylate salt/co-crystal of Compound I.
**FIG. 9** shows a thermogravimetric analysis (TGA) of a mesylate salt/co-crystal of Compound I.

### Definitions

As used herein, "treatment" or "treating" refers to an amelioration of a disease or disorder, or at least one discernible symptom thereof. In another embodiment, "treatment" or "treating" refers to an amelioration of at least one measurable physical parameter, not necessarily discernible by the patient. In yet another embodiment, "treatment" or "treating" refers to inhibiting the progression of a disease or disorder, either physically, e.g., stabilization of a discernible symptom, physiologically, e.g., stabilization of a physical parameter, or both. In yet another embodiment, "treatment" or "treating" refers to delaying the onset of a disease or disorder.

By "optional" or "optionally" is meant that the subsequently described event or circumstance may or may not occur, and that the description includes instances where the event or circumstance occurs and instances in which is does not. For example, "optionally substituted aryl" encompasses both "aryl" and "substituted aryl" as defined below. It will be understood by those skilled in the art, with respect to any group containing one or more substituents, that such groups are not intended to introduce any substitution or substitution patterns that are sterically impractical, synthetically non-feasible and/or inherently unstable.

As used herein, the term "hydrate" refers to a crystal form with either a stoichiometric or non-stoichiometric amount of water is incorporated into the crystal structure.

The term "alkenyl" as used herein refers to an unsaturated straight or branched hydrocarbon having at least one carbon-carbon double bond, such as a straight or branched group of 2-8 carbon atoms, referred to herein as (C₂₋C₈) alkenyl. Exemplary alkenyl groups include, but are not limited to, vinyl, allyl, butenyl, pentenyl, hexenyl, butadienyl, pentadienyl, hexadienyl, 2-ethylhexenyl, 2-propyl-2-butenyl, and 4-(2-methyl-3-butene)-pentenyl.

The term "alkoxy" as used herein refers to an alkyl group attached to an oxygen (-O-alkyl-). "Alkoxy" groups also include an alkenyl group attached to an oxygen ("alkenyloxy") or an alkynyl group attached to an oxygen ("alkynyloxy") groups. Exemplary alkoxy groups include, but are not limited to, groups with an alkyl, alkenyl or alkynyl group of 1-8 carbon atoms, referred to herein as (C₁₋C₈) alkoxy. Exemplary alkoxy groups include, but are not limited to, methoxy and ethoxy.

The term "alkyl" as used herein refers to a saturated straight or branched hydrocarbon, such as a straight or branched group of 1-8 carbon atoms, referred to herein as (C₁₋C₈) alkyl. Exemplary alkyl groups include, but are not limited to, methyl, ethyl, propyl, isopropyl, 2-methyl-1-propyl, 2-methyl-2-propyl, 2-methyl-1-butyl, 3-methyl-1-butyl, 2-methyl-3-butyl, 2,2-dimethyl-1-propyl, 2-methyl-1-pentyl, 3-methyl-1-pentyl, 4-methyl-1-pentyl, 2-methyl-2-pentyl, 3-methyl-2-pentyl, 4-methyl-2-pentyl, 2,2-dimethyl-1-butyl, 3,3-dimethyl-1-butyl, 2-ethyl-1-butyl, butyl, isobutyl, t-butyl, pentyl, isopentyl, neopentyl, hexyl, heptyl, and octyl.

The term "amide" as used herein refers to -NRₐC(O)(R_{b})- or -C(O)NR_{b}R_{c}, wherein Rₐ, R_{b} and R_{c} are each independently selected from alkyl, alkenyl, alkynyl, aryl, arylalkyl, cycloalkyl, haloalkyl, heteroaryl, heterocyclyl, and hydrogen. The amide can be attached to another group through the carbon, the nitrogen, R_{b}, or R_{c}. The amide also may be cyclic, for example R_{b} and R_{c}, may be joined to form a 3- to 8-membered ring, such as 5- or 6-membered ring. The term "amide" encompasses groups such as sulfonamide, urea, ureido, carbamate, carbamic acid, and cyclic versions thereof. The term "amide" also encompasses an amide group attached to a carboxy group, e.g., -amide-COOH or salts such as -amide-COONa, an amino group attached to a carboxy group (e.g., -amino-COOH or salts such as -amino-COONa).

The term "amine" or "amino" as used herein refers to the form -NR_{d}Rₑ or -N(R_{d})Rₑ-, where R_{d} and Rₑ are independently selected from alkyl, alkenyl, alkynyl, aryl, arylalkyl, carbamate, cycloalkyl, haloalkyl, heteroaryl, heterocycle, and hydrogen. The amino can be attached to the parent molecular group through the nitrogen. The amino also may be cyclic, for example any two of R_{d} and Rₑ may be joined together or with the N to form a 3- to 12-membered ring (e.g., morpholino or piperidinyl). The term amino also includes the corresponding quaternary ammonium salt of any amino group. Exemplary amino groups include alkylamino groups, wherein at least one of R_{d} or Rₑ is an alkyl group. In some embodiments Rd and Re each may be optionally substituted with hydroxyl, halogen, alkoxy, ester, or amino.

The term "aryl" as used herein refers to a mono-, bi-, or other multi-carbocyclic, aromatic ring system. The aryl group can optionally be fused to one or more rings selected from aryls, cycloalkyls, and heterocyclyls. The aryl groups of this present disclosure can be substituted with groups selected from alkoxy, aryloxy, alkyl, alkenyl, alkynyl, amide, amino, aryl, arylalkyl, carbamate, carboxy, cyano, cycloalkyl, ester, ether, formyl, halogen, haloalkyl, heteroaryl, heterocyclyl, hydroxyl, ketone, nitro, phosphate, sulfide, sulfinyl, sulfonyl, sulfonic acid, sulfonamide, and thioketone. Exemplary aryl groups include, but are not limited to, phenyl, tolyl, anthracenyl, fluorenyl, indenyl, azulenyl, and naphthyl, as well as benzo-fused carbocyclic moieties such as 5,6,7,8-tetrahydronaphthyl. Exemplary aryl groups also include, but are not limited to, a monocyclic aromatic ring system, wherein the ring comprises 6 carbon atoms, referred to herein as "(C₆) aryl."

The term "arylalkyl" as used herein refers to an alkyl group having at least one aryl substituent (e.g., -aryl-alkyl-). Exemplary arylalkyl groups include, but are not limited to, arylalkyls having a monocyclic aromatic ring system, wherein the ring comprises 6 carbon atoms, referred to herein as "(C₆) arylalkyl."

The term "carbamate" as used herein refers to the form -R_{g}OC(O)N(Rₕ)-, -R_{g}OC(O)N(Rₕ)Rᵢ-, or -OC(O)NRₕRᵢ, wherein R_{g}, Rₕ and Rᵢ are each independently selected from alkyl, alkenyl, alkynyl, aryl, arylalkyl, cycloalkyl, haloalkyl, heteroaryl, heterocyclyl, and hydrogen. Exemplary carbamates include, but are not limited to, arylcarbamates or heteroaryl carbamates (e.g., wherein at least one of R_{g}, Rₕ and Rᵢ are independently selected from aryl or heteroaryl, such as pyridine, pyridazine, pyrimidine, and pyrazine).

The term "carbocycle" as used herein refers to an aryl or cycloalkyl group.

The term "carboxy" as used herein refers to -COOH or its corresponding carboxylate salts (e.g., -COONa). The term carboxy also includes "carboxycarbonyl," e.g. a carboxy group attached to a carbonyl group, e.g., -C(O)-COOH or salts, such as -C(O)-COONa.

The term "cycloalkoxy" as used herein refers to a cycloalkyl group attached to an oxygen.

The term "cycloalkyl" as used herein refers to a saturated or unsaturated cyclic, bicyclic, or bridged bicyclic hydrocarbon group of 3-12 carbons, or 3-8 carbons, referred to herein as "(C₃-C₈) cycloalkyl," derived from a cycloalkane. Exemplary cycloalkyl groups include, but are not limited to, cyclohexanes, cyclohexenes, cyclopentanes, and cyclopentenes. Cycloalkyl groups may be substituted with alkoxy, aryloxy, alkyl, alkenyl, alkynyl, amide, amino, aryl, arylalkyl, carbamate, carboxy, cyano, cycloalkyl, ester, ether, formyl, halogen, haloalkyl, heteroaryl, heterocyclyl, hydroxyl, ketone, nitro, phosphate, sulfide, sulfinyl, sulfonyl, sulfonic acid, sulfonamide and thioketone. Cycloalkyl groups can be fused to other cycloalkyl saturated or unsaturated, aryl, or heterocyclyl groups.

The term "dicarboxylic acid" as used herein refers to a group containing at least two carboxylic acid groups such as saturated and unsaturated hydrocarbon dicarboxylic acids and salts thereof. Exemplary dicarboxylic acids include alkyl dicarboxylic acids. Dicarboxylic acids may be substituted with alkoxy, aryloxy, alkyl, alkenyl, alkynyl, amide, amino, aryl, arylalkyl, carbamate, carboxy, cyano, cycloalkyl, ester, ether, formyl, halogen, haloalkyl, heteroaryl, heterocyclyl, hydrogen, hydroxyl, ketone, nitro, phosphate, sulfide, sulfinyl, sulfonyl, sulfonic acid, sulfonamide and thioketone. Dicarboxylic acids include, but are not limited to succinic acid, glutaric acid, adipic acid, suberic acid, sebacic acid, azelaic acid, maleic acid, phthalic acid, aspartic acid, glutamic acid, malonic acid, fumaric acid, (+)/(-)-malic acid, (+)/(-) tartaric acid, isophthalic acid, and terephthalic acid. Dicarboxylic acids further include carboxylic acid derivatives thereof, such as anhydrides, imides, hydrazides (for example, succinic anhydride and succinimide).

The term "ester" refers to the structure -C(O)O-, -C(O)O-Rⱼ₋, -RₖC(O)O-Rⱼ₋, or -RₖC(O)O-, where O is not bound to hydrogen, and Rⱼ and Rₖ can independently be selected from alkoxy, aryloxy, alkyl, alkenyl, alkynyl, amide, amino, aryl, arylalkyl, cycloalkyl, ether, haloalkyl, heteroaryl, and heterocyclyl. Rₖ can be a hydrogen, but Rⱼ cannot be hydrogen. The ester may be cyclic, for example the carbon atom and Rⱼ, the oxygen atom and Rₖ, or Rⱼ and Rₖ may be joined to form a 3- to 12-membered ring. Exemplary esters include, but are not limited to, alkyl esters wherein at least one of Rj or Rk is alkyl, such as -O-C(O)-alkyl, -C(O)-O-alkyl-, and -alkyl-C(O)-O-alkyl-. Exemplary esters also include aryl or heteoraryl esters, e.g. wherein at least one of Rj or Rk is a heteroaryl group such as pyridine, pyridazine, pyrimidine and pyrazine, such as a nicotinate ester. Exemplary esters also include reverse esters having the structure -RₖC(O)O-, where the oxygen is bound to the parent molecule. Exemplary reverse esters include succinate, D-argininate, L-argininate, L-lysinate and D-lysinate. Esters also include carboxylic acid anhydrides and acid halides.

The terms "halo" or "halogen" as used herein refer to F, Cl, Br, or I.

The term "haloalkyl" as used herein refers to an alkyl group substituted with one or more halogen atoms. "Haloalkyls" also encompass alkenyl or alkynyl groups substituted with one or more halogen atoms.

The term "heteroaryl" as used herein refers to a mono-, bi-, or multi-cyclic, aromatic ring system containing one or more heteroatoms, for example 1-3 heteroatoms, such as nitrogen, oxygen, and sulfur. Heteroaryls can be substituted with one or more substituents including alkoxy, aryloxy, alkyl, alkenyl, alkynyl, amide, amino, aryl, arylalkyl, carbamate, carboxy, cyano, cycloalkyl, ester, ether, formyl, halogen, haloalkyl, heteroaryl, heterocyclyl, hydroxyl, ketone, nitro, phosphate, sulfide, sulfinyl, sulfonyl, sulfonic acid, sulfonamide and thioketone. Heteroaryls can also be fused to non-aromatic rings. Illustrative examples of heteroaryl groups include, but are not limited to, pyridinyl, pyridazinyl, pyrimidyl, pyrazyl, triazinyl, pyrrolyl, pyrazolyl, imidazolyl, (1,2,3)- and (1,2,4)-triazolyl, pyrazinyl, pyrimidilyl, tetrazolyl, furyl, thienyl, isoxazolyl, thiazolyl, furyl, phenyl, isoxazolyl, and oxazolyl. Exemplary heteroaryl groups include, but are not limited to, a monocyclic aromatic ring, wherein the ring comprises 2-5 carbon atoms and 1-3 heteroatoms, referred to herein as "(C₂-C₅) heteroaryl."

The terms "heterocycle," "heterocyclyl," or "heterocyclic" as used herein refer to a saturated or unsaturated 3-, 4-, 5-, 6- or 7-membered ring containing one, two, or three heteroatoms independently selected from nitrogen, oxygen, and sulfur. Heterocycles can be aromatic (heteroaryls) or non-aromatic. Heterocycles can be substituted with one or more substituents including alkoxy, aryloxy, alkyl, alkenyl, alkynyl, amide, amino, aryl, arylalkyl, carbamate, carboxy, cyano, cycloalkyl, ester, ether, formyl, halogen, haloalkyl, heteroaryl, heterocyclyl, hydroxyl, ketone, nitro, phosphate, sulfide, sulfinyl, sulfonyl, sulfonic acid, sulfonamide and thioketone. Heterocycles also include bicyclic, tricyclic, and tetracyclic groups in which any of the above heterocyclic rings is fused to one or two rings independently selected from aryls, cycloalkyls, and heterocycles. Exemplary heterocycles include acridinyl, benzimidazolyl, benzofuryl, benzothiazolyl, benzothienyl, benzoxazolyl, biotinyl, cinnolinyl, dihydrofuryl, dihydroindolyl, dihydropyranyl, dihydrothienyl, dithiazolyl, furyl, homopiperidinyl, imidazolidinyl, imidazolinyl, imidazolyl, indolyl, isoquinolyl, isothiazolidinyl, isothiazolyl, isoxazolidinyl, isoxazolyl, morpholinyl, oxadiazolyl, oxazolidinyl, oxazolyl, piperazinyl, piperidinyl, pyranyl, pyrazolidinyl, pyrazinyl, pyrazolyl, pyrazolinyl, pyridazinyl, pyridyl, pyrimidinyl, pyrimidyl, pyrrolidinyl, pyrrolidin-2-onyl, pyrrolinyl, pyrrolyl, quinolinyl, quinoxaloyl, tetrahydrofuryl, tetrahydroisoquinolyl, tetrahydropyranyl, tetrahydroquinolyl, tetrazolyl, thiadiazolyl, thiazolidinyl, thiazolyl, thienyl, thiomorpholinyl, thiopyranyl, and triazolyl.

The terms "hydroxy" and "hydroxyl" as used herein refer to -OH.

The term "hydroxyalkyl" as used herein refers to a hydroxy attached to an alkyl group.

The term "hydroxyaryl" as used herein refers to a hydroxy attached to an aryl group.

The term "ketone" as used herein refers to the structure -C(O)-Rn (such as acetyl, -C(O)CH₃) or -Rₙ₋C(O)-Rₒ₋. The ketone can be attached to another group through Rₙ or Rₒ. Rₙ or Rₒ can be alkyl, alkenyl, alkynyl, cycloalkyl, heterocyclyl or aryl, or Rₙ or Rₒ can be joined to form a 3- to 12-membered ring.

The term "phenyl" as used herein refers to a 6-membered carbocyclic aromatic ring. The phenyl group can also be fused to a cyclohexane or cyclopentane ring. Phenyl can be substituted with one or more substituents including alkoxy, aryloxy, alkyl, alkenyl, alkynyl, amide, amino, aryl, arylalkyl, carbamate, carboxy, cyano, cycloalkyl, ester, ether, formyl, halogen, haloalkyl, heteroaryl, heterocyclyl, hydroxyl, ketone, phosphate, sulfide, sulfinyl, sulfonyl, sulfonic acid, sulfonamide and thioketone.

The term "thioalkyl" as used herein refers to an alkyl group attached to a sulfur (-S-alkyl-).

"Alkyl," "alkenyl," "alkynyl", "alkoxy", "amino" and "amide" groups can be optionally substituted with or interrupted by or branched with at least one group selected from alkoxy, aryloxy, alkyl, alkenyl, alkynyl, amide, amino, aryl, arylalkyl, carbamate, carbonyl, carboxy, cyano, cycloalkyl, ester, ether, formyl, halogen, haloalkyl, heteroaryl, heterocyclyl, hydroxyl, ketone, phosphate, sulfide, sulfinyl, sulfonyl, sulfonic acid, sulfonamide, thioketone, ureido and N. The substituents may be branched to form a substituted or unsubstituted heterocycle or cycloalkyl.

As used herein, a suitable substitution on an optionally substituted substituent refers to a group that does not nullify the synthetic or pharmaceutical utility of the compounds of the present disclosure or the intermediates useful for preparing them. Examples of suitable substitutions include, but are not limited to: C₁₋₈ alkyl, alkenyl or alkynyl; C₁₋₆ aryl, C₂₋₅ heteroaryl; C₃₇ cycloalkyl; C₁₋₈ alkoxy; C₆ aryloxy; -CN; -OH; oxo; halo, carboxy; amino, such as -NH(C₁₋₈ alkyl), -N(C₁₋₈ alkyl)₂, -NH((C₆)aryl), or -N((C₆)aryl)₂; formyl; ketones, such as -CO(C₁₋₈ alkyl), -CO((C₆ aryl) esters, such as -CO₂(C₁₋₈ alkyl) and -CO₂ (C₆ aryl). One of skill in art can readily choose a suitable substitution based on the stability and pharmacological and synthetic activity of the compound of the present disclosure.

The term "pharmaceutically acceptable composition" as used herein refers to a composition comprising at least one compound as disclosed herein formulated together with one or more pharmaceutically acceptable carriers.

The term "pharmaceutically acceptable carrier" as used herein refers to any and all solvents, dispersion media, coatings, isotonic and absorption delaying agents, and the like, that are compatible with pharmaceutical administration. The use of such media and agents for pharmaceutically active substances is well known in the art. The compositions may also contain other active compounds providing supplemental, additional, or enhanced therapeutic functions.

As summarized above, ER+ breast cancer may be treated with a combination therapy comprising administration of a BET bromodomain inhibitor of Formula la or Formula Ib, or a stereo isomer, tautomer, or pharmaceutically acceptable salt/co-crystal thereof, and a second therapeutic agent to a subject in need thereof: 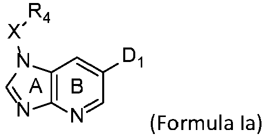 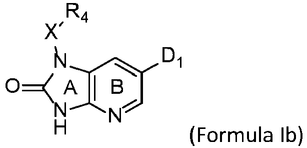 wherein:
**Ring A** and **Ring B** may be optionally substituted with groups independently selected from hydrogen, deuterium, -NH₂, amino, heterocycle(C₄-C₆), carbocycle(C₄-C₆), halogen, -CN, - OH, -CF₃, alkyl (C₁-C₆), thioalkyl (C₁-C₆), alkenyl (C₁-C₆), and alkoxy (C₁-C₆);
**X** is selected from -NH-, -CH₂-, -CH₂CH₂-, -CH₂CH₂CH₂-, -CH₂CH₂O-, -CH₂CH₂NH-, - CH₂CH₂S-, -C(O)-, -C(O)CH₂-, -C(O)CH₂CH₂-, -CH₂C(O)-, -CH₂CH₂C(O)-, -C(O)NH-, -C(O)O-, -C(O)S-, -C(O)NHCH₂-, -C(O)OCH₂-, -C(O)SCH₂-, wherein one or more hydrogen may independently be replaced with deuterium, hydroxyl, methyl, halogen, -CF₃, ketone, and where S may be oxidized to sulfoxide or sulfone;
**R₄** is selected from optionally substituted 3-7 membered carbocycles and heterocycles; and
**D₁** is selected from the following 5-membered monocyclic heterocycles: 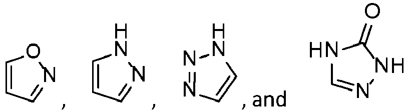 which are optionally substituted with hydrogen, deuterium, alkyl (C₁-C₄), alkoxy (C₁-C₄), amino, halogen, amide, -CF₃, -CN, -N₃, ketone (C₁-C₄), -S(O)Alkyl(C₁-C₄), -SO₂alkyl(C₁-C₄), -thioalkyl(C₁-C₄), -COOH, and/or ester, each of which may be optionally substituted with hydrogen, F, Cl, Br, -OH, -NH₂, -NHMe, -OMe, -SMe, oxo, and/or thio-oxo.

Compounds of Formula la and Ib, including Compound I, have been previously described in International Patent Publication WO 2015/002754,
and particularly its description of the compounds of Formula la and Formula Ib, including Compound I, their synthesis, and the demonstration of their BET bromodomain inhibitor activity.

The invention relates to treating ER+ breast cancer comprising administrating to a subject in need thereof, a compound selected from 1-benzyl-6-(3,5-dimethylisoxazol-4-yl)-N-methyl-1H-imidazo[4,5-b]pyridin-2-amine (Compound I) and pharmaceutically acceptable salts or co-crystals thereof, concomitantly with another therapeutic agent, as defined in the claims.

In one embodiment, the BET bromodomain inhibitor administered in the invention is the mesylate salt or co-crystal of 1-benzyl-6-(3,5-dimethylisoxazol-4-yl)-N-methyl-1H-imidazo[4,5-b]pyridin-2-amine (Compound I).

In one embodiment, a compound selected from 1-benzyl-6-(3,5-dimethylisoxazol-4-yl)-N-methyl-1H-imidazo[4,5-b]pyridin-2-amine (Compound I) and pharmaceutically acceptable salts or co-crystals thereof, is dosed with a second therapeutic agent without resulting in thrombocytopenia as a dose-limiting toxicity.

A suitable second therapeutic agent may be a selective-estrogen receptor degrader (SERD). Accordingly, in some embodiments, the second therapeutic agent is fulvestrant.

In some embodiments, the subject has previously been treated with an aromatase inhibitor.

In some embodiments according to the invention, the second therapeutic agent is fulvestrant.

A suitable second therapeutic agent may be a CDK4/6 inhibitor. Accordingly, in some embodiments according to the invention, the second therapeutic agent is abemaciclib.

In some embodiments, the second therapeutic agent is abemaciclib.

In some embodiments, the subject previously has been treated with a breast cancer therapy. In some embodiments, the prior breast cancer therapy is chemotherapy. In some embodiments, the prior breast cancer therapy is treatment with a CDK4/6 inhibitor. In some embodiments, the prior breast cancer therapy is immunotherapy.

In some embodiments, the subject is a human.

In some embodiments, the BET bromodomain inhibitor as described herein is administered concomitantly with the second therapeutic agent. "Concomitantly" as used herein means that the BET bromodomain inhibitor and the second therapeutic agent are administered with a time separation of a few seconds (for example 15 sec., 30 sec., 45 sec., 60 sec. or less), several minutes (for example 1 min., 2 min., 5 min. or less, 10 min. or less, 15 min. or less), or 1-12 hours. When administered concomitantly, the BET bromodomain inhibitor and the other therapeutic agent may be administered in two or more administrations, and contained in separate compositions or dosage forms, which may be contained in the same or different package or packages.

### LIST OF REFERENCES

Chou, T.C. and Talalay, P. (2984), Quantitative analysis of dose-effect relationships: The combined effects of multiple drugs or enzyme inhibitors. Advances in Enzyme Regulation 22, 27-55.
Feng, Q., Zhang, Z., Shea, M.J., Creighton, C.J., Coarfa, C., Hilsenbeck, S.G., Lanz, R., He, B., Wang, L., Fu, X., et al. (2014). An epigenomic approach to therapy for tamoxifen-resistant breast cancer. Cell Res 24, 809-819.
Ladd, B., Mazzola, A.M., Bihani, T., Lai, Z., Bradford, J., Collins, M., Barry, E., Goeppert, A.U., Weir, H.M., Hearne, K., et al. (2016). Effective combination therapies in preclinical endocrine resistant breast cancer models harboring ER mutations. Oncotarget 7, 54120-54136.
Nagarajan, S., Hossan, T., Alawi, M., Najafova, Z., Indenbirken, D., Bedi, U., Taipaleenmaki, H., Ben-Batalla, I., Scheller, M., Loges, S., et al. (2014). Bromodomain Protein BRD4 Is Required for Estrogen Receptor-Dependent Enhancer Activation and Gene Transcription. Cell reports 8, 460-469.
Sengupta, S., Biarnes, M., Clarke, R., and Jordan, V.C. (2015). Inhibition of BET proteins impairs estrogen-mediated growth and transcription in breast cancers by pausing RNA polymerase advancement. Breast Cancer Res Treat 150, 265-278.

### EXAMPLES

Tissue culture media and reagents were obtained from ThermoFisher Scientific. Fulvestrant was obtained from Sigma, abemaciclib and palbociclib were obtained from Selleckchem.

### Example 1: Synthesis of Compound 1

### Step A: Synthesis of 5-Bromo-N³-(phenylmethylene)pyridine-2,3-diamine (Compound B)

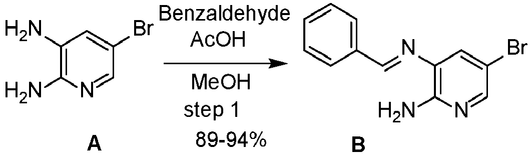

Starting material A was dissolved in methanol and acetic acid. The solution was cooled to 0-5 °C and benzaldehyde was added dropwise. Once the reaction was complete, process water and a NaHCO₃ solution was added dropwise, keeping the temperature low (0-5 °C). The solid was filtered off and washed with methanol/water 1:1, followed by drying, leaving Compound B in 94% yield and +99% purity by HPLC. ¹H-NMR (DMSO-d₆): δ 8.75 (1H), 8.04 (2H), 7.93 (1H), 7.65 (1H), 7.50-7.60 (3H).

### Step B: Synthesis of N³-Benzyl-5-bromopyridine-2,3-diamine (Compound C)

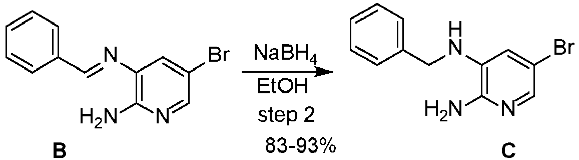

Compound **B** was dissolved in ethanol and NaHB₄ was added in portions keeping the temperature between 15-25 °C. The reaction mixture was stirred for 8-15 h until the reaction was complete as monitored by HPLC. A HCl solution was added, adjusting pH to 6-7, followed by process water, keeping the temperature between 15-25 °C. The mixture was stirred for 1-5 h, filtered and washed with an ethanol/water mixture. Following drying at ~60 °C for 15-20 h, Compound **C** was obtained. ¹H-NMR (DMSO-d₆): d 7.2-7.4 (6 H), 6.55 (1 H), 5.70-5.83 (3 H), 4.30 (2 H).

### Step C: Synthesis of N³-Benzyl-5-(3,5-dimethyl-1,2-oxazol-4-yl)pyridine-2,3-diamine (Compound D)

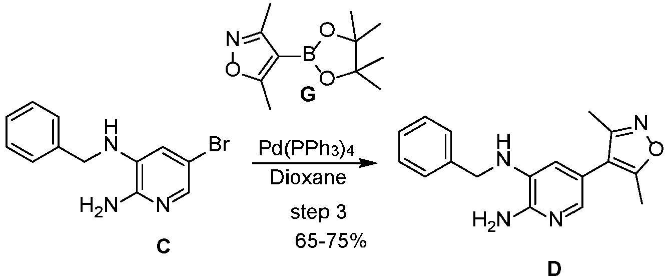

Compound **C,** Compound **G,** and potassium phosphate tribasic trihydrate were mixed followed by addition of 1,4-Dioxane and process water. The resulting mixture was thoroughly purged with nitrogen. Tetrakis(triphenylphosphine)palladium(0) was added and the mixture was heated to ≥90°C until the ratio of Compound **C** to Compound **D** was not more than 1%. After cooling, the reaction mixture was filtered, the solid washed with 1,4-dioxane and then concentrated. Process water was added and the mixture was stirred until the amount of Compound **D** remaining in the mother liquors was not more than 0.5%. Compound **D** was isolated by filtration and sequentially washed with 1,4-dioxane/water and t-butylmethyl ether. The wet cake was mixed in methylene chloride and silica gel. After stirring, the mixture was filtered then concentrated. The mixture was cooled and t-butylmethyl ether was added. The product was isolated by filtration and dried until the methylene chloride, t-butylmethyl ether, and moisture levels are not more than 0.5%. ¹H-NMR (DMSO-d₆): δ 7.30-7.45 (4 H), 7.20-7.25 (2 H), 6.35 (1 H), 5.65-5.80 (3 H), 4.30-4.40 (2 H), 2.15 (3 H), 1.95 (3 H).

### Step D: Synthesis of 1-Benzyl-6-(3,5-dimethyl-1,2-oxazol-4-yl)-3H-imidazo[4,5-b]pyridin-2-one (Compound E)

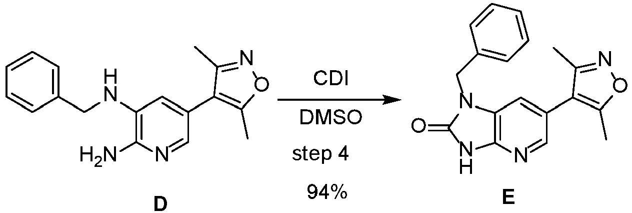

Carbonyldiimidazole solid was added to a stirring mixture of Compound **D** and dimethylsulfoxide. The mixture was heated until the ratio of Compound **D** to Compound **E** was NMT 0.5%. The mixture was cooled and process water was added over several hours. The resulting mixture was stirred at ambient temperature for at least 2 h. The product was isolated by filtration and washed with process water. The dimethylsulfoxide was verified to be NMT 0.5% before drying using heat and vacuum. Drying was complete when the moisture level was NMT 0.5%, leaving Compound E. ¹H-NMR (DMSO-d₆): δ 11.85 (1 H), 7.90 (1 H), 7.20-7.45 (6 H), 5.05 (2 H), 3.57 (3 H), 2.35 (3 H), 2.15 (3 H).

### Step E: Synthesis of 4-[1-Benzyl-2-chloro-1H-imidazo[4,5-b]pyridine-6-yl]-3,5-dimethyl-1,2-oxazole (Compound F)

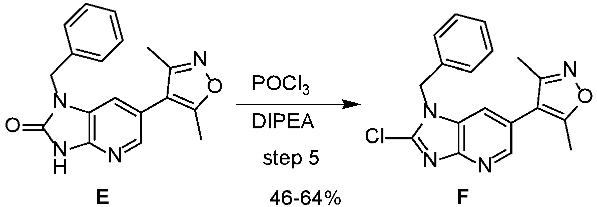

Compound E and phosphorus oxychloride were mixed and then treated with diisopropylethyl amine (DIPEA), which can be added dropwise. The resulting mixture was heated for several hours, cooled, and sampled for reaction completion. If the ratio of Compound E to Compound F was not more than 0.5% then the reaction was complete. Otherwise, the reaction was heated for additional time and sampled as before. After the reaction was complete, the mixture was concentrated then cooled. Ethyl acetate was added and the mixture was concentrated under vacuum several times. Ethyl acetate (EtOAc) was added to the concentrate, the mixture was cooled and then added to aqueous sodium bicarbonate. The organic phase was separated and the organic layer was washed with aqueous sodium bicarbonate and then water. The organic phase was concentrated, ethyl acetate was added, and the mixture was concentrated to assure that the moisture level was not more than 0.2%. The mixture in ethyl acetate was decolorized with carbon. The mixture was concentrated and n-heptane was added. The product was isolated by filtration and dried under vacuum. Drying was complete when residual moisture, ethyl acetate, and n-heptane were not more than 0.5%. ¹H-NMR (MeOH-d₄): δ 8.40 (1 H), 7.90 (1 H), 7.25-7.45 (5 H), 5.65 (2 H), 2.37 (3 H), 2.22 (3 H).

### Step F: Synthesis of 1-benzyl-6-(3,5-dimethyl-1,2-oxazol-4-yl)-N-methyl-1H-imidazo[4,5-b]pyridine-2-amine (Compound I)

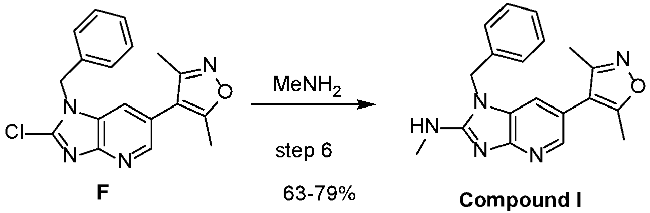

Compound **F** was mixed with methylamine in tetrahydrofuran (THF) and stirred at ambient temperature until the ratio of Compound **F** to Compound I was NMT 0.1% by HPLC. After reaction completion, the mixture was concentrated under vacuum, process water added, and the product isolated by filtration. The filter cake was washed with process water. The wet cake was dissolved in hydrochloric acid and the resulting solution was washed with methylene chloride to remove impurities. The aqueous solution was neutralized with a sodium hydroxide solution and Compound I was isolated by filtration, washed with process water, and dried under vacuum. If necessary, to remove any remaining hydrochloric acid, the dried material can be dissolved in ethanol, treated with a solution of sodium hydroxide in ethanol, followed by addition of process water to precipitate the product. Compound I was isolated by filtration, washed with process water, and dried. ¹H-NMR (DMSO-d₆): δ 7.96 (d, 1H, J=2.0 Hz), 7.42 (d, 1H, J=2.0 Hz), 7.37 (q, 1H, J=4.2 Hz), 7.32 (m, 2H), 7.26 (m, 1H), 7.24 (m, 2H), 5.30 (s, 2H), 3.00 (d, 3H, 4.5 Hz), 2.34 (s, 3H), 2.16 (s, 3H). ¹³C-NMR (DMSO-d₆): δ 164.8, 158.4, 157.7, 156.0, 141.1, 136.4, 128.6 (2C), 127.5, 127.4, 127.2 (2C), 115.8, 114.2 (2C), 44.5, 29.3, 11.2, 10.3.

### Example 2: Crystalline mesylate of Compound I

About 5 g of Compound I was dissolved in ethanol (115 mL) and a solution of methanesulfonic acid in ethanol (10 mL, 158.7 mg/mL) was added, according to a 1:1 molar ratio. The mixture was shaken at 50 °C for 2 h before concentrated to half volume and stirred overnight. The formed solid (mesylate salt/co-crystal of Compound I Form I) was isolated, dried, and characterized.

The mesylate salt/co crystal of Compound I Form I was also obtained from other solvents and solvent mixtures, including acetone and acetonitrile.

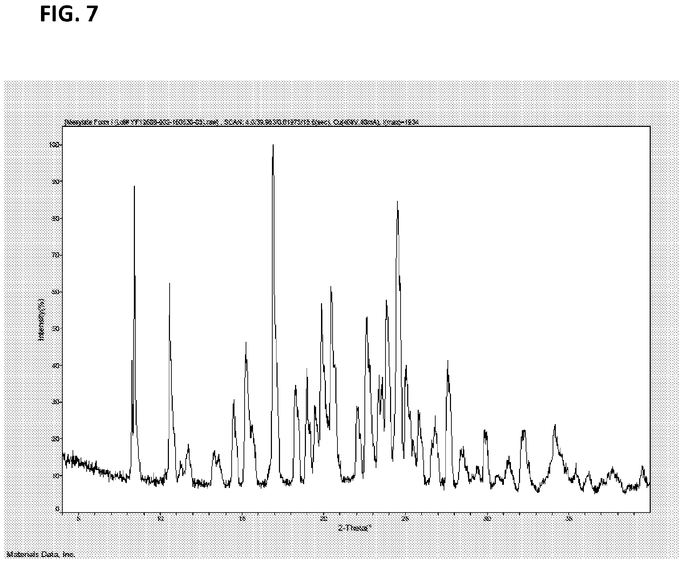

The mesylate salt/co crystal of Compound I Form I was characterized by XRPD comprising the following peaks, in terms of 2-theta, at 8.4 ± 0.2, 10.6 ± 0.2, 11.7 ± 0.2, 14.5 ± 0.2, 15.3 ± 0.2, 16.9 ± 0.2, 18.2 ± 0.2, 19.0 ± 0.2, 19.9 ± 0.2, 20.5 ± 0.2, 22.6 ± 0.2, 23.8 ± 0.2, 24.5 ± 0.2, and 27.6± 0.2 degrees, as determined on a diffractometer using Cu-K_{α} radiation tube (FIG. 7).

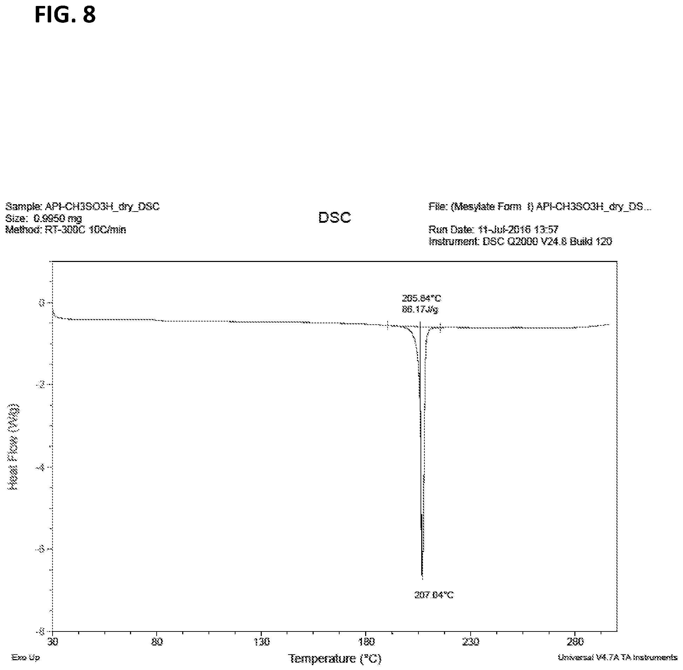

The mesylate salt/co crystal of Compound I Form I was characterized by DSC having an endothermic peak at a temperature of about 207 °C (FIG. 8).

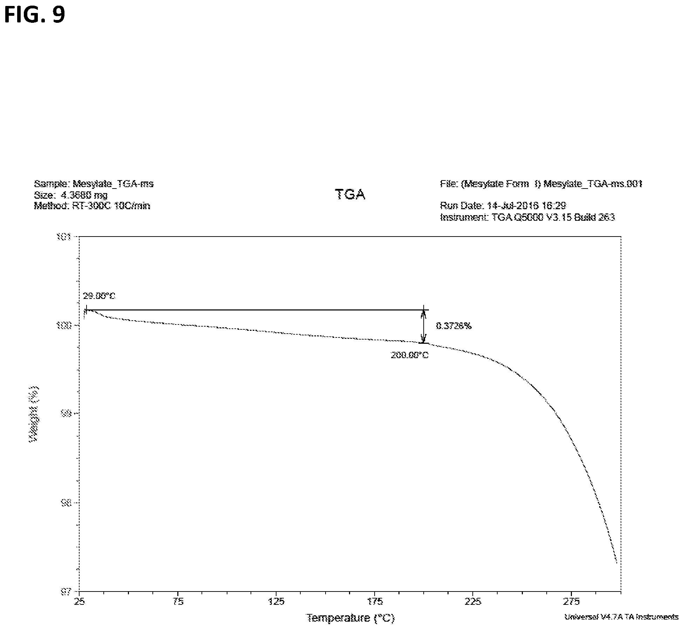

The mesylate salt/co crystal of Compound I Form I was characterized by TGA, having a thermogram as shown in FIG. 9, confirming that Compound I Form I is an anhydrous form.

### Example 3: Synergistic inhibition of ER+ breast cancer cell line viability by combination of Compound I with abemaciclib

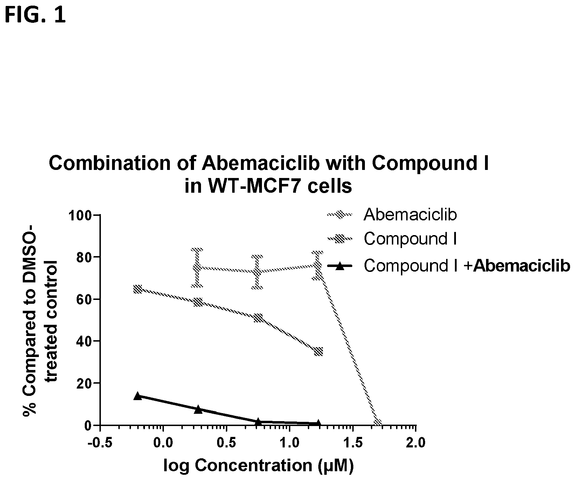

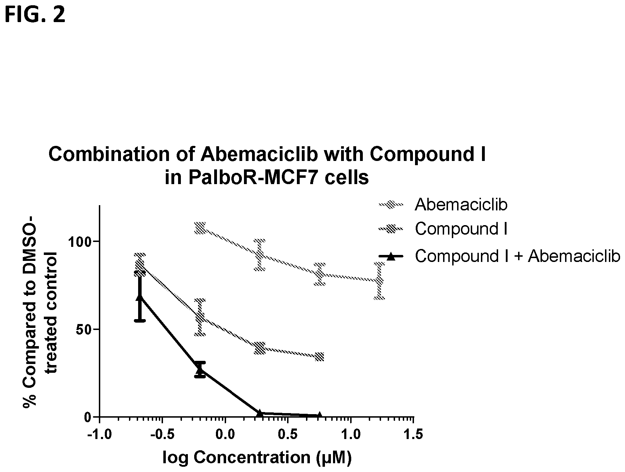

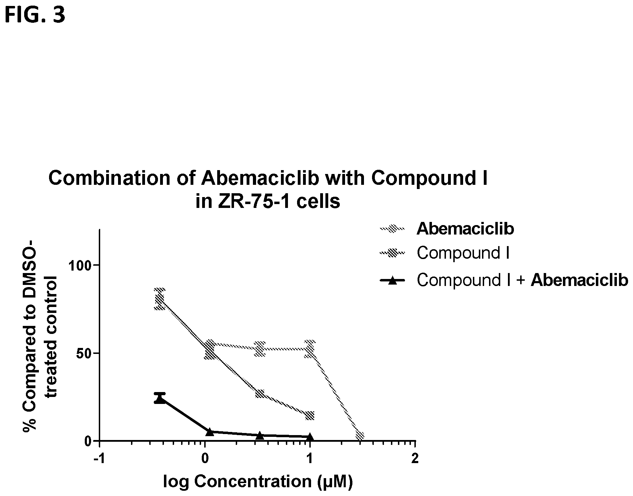

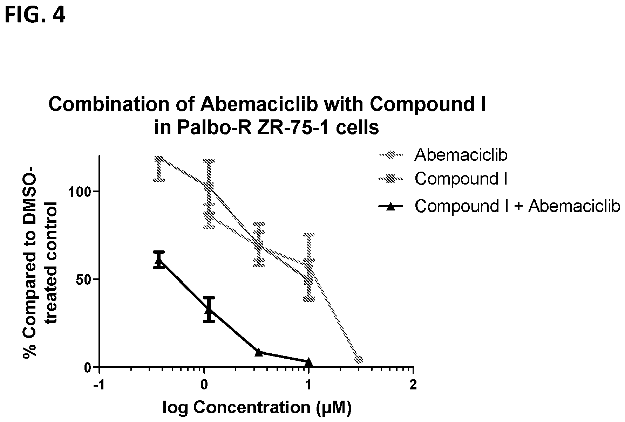

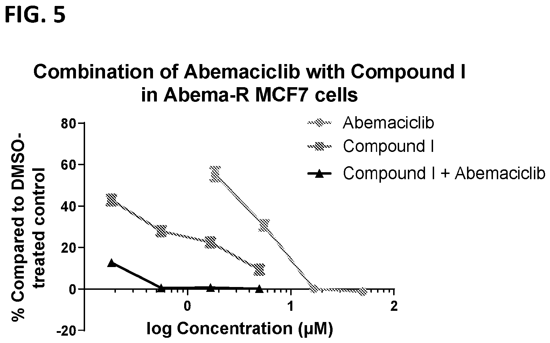

MCF7, Palbo-R-MCF7, ZR-75-1, Abema-R MCF7, and Palbo-R ZR-75-1 cells were plated at a density of 7,500 cells per well in 96 well flat bottom plates in 1640-RPMI media containing 10% FBS and penicillin/streptomycin and incubated for 24 hours at 37 °C, 5% CO₂. Media was replaced with 1640-RPMI containing 10% FBS with constant ratios of either Compound I or abemaciclib as single agents, or a combination of both drugs at four different concentrations (2X IC50, 1X IC50, 0.5X IC50, 0.25X IC50), and incubated at 37 °C, 5% CO₂ for 7 days. The cells were retreated as described above on the 3rd or 4th day. Triplicate wells were used for each concentration and wells containing only media with 0.1% DMSO were used as a control. To measure cell viability, 100 uL of a 1:100 dilution of GF-AFC substrate into the Assay Buffer (CellTiter Fluor Cell Viability Assay (Promega)) were added to each well and incubated at 37°C, 5% CO₂ for an additional 30-90 minutes. Fluorescence at 380-400 nm Excitation/505 nm Emission was read in a fluorometer and the percentage of cell titer relative to DMSO-treated cells was calculated after correcting for background by subtracting the blank well's signal. IC50 values for single agents were calculated using the GraphPad Prism software. Quantification of synergy was done by calculating combination indices (CI) using the CalcuSyn software (Biosoft) based on the Chou-Talalay algorithm (Chou and Talalay, 1984), and averaging the Cl values for the effective doses (ED) 50, 75, and 90. As shown in FIGs. 1-5, addition of Compound I to abemaciclib resulted in improved inhibition of cell viability compared to either single agent with an average Cl value of 0.08-0.35 depending upon the cell line.

### Example 4: Synergistic inhibition of MCF7 cell viability by combination of Compound I with fulvestrant

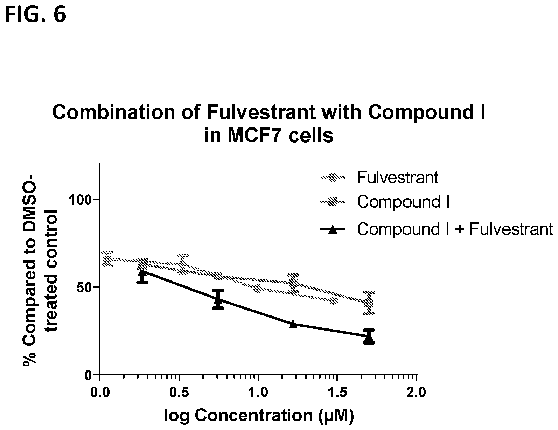

MCF7 cells were plated at a density of 7,500 cells per well in 96 well flat bottom plates in phenol-red free 1640-RPMI media containing 10% charcoal-stripped FBS and penicillin/streptomycin and incubated for 24 hours at 37 °C, 5% CO₂. Media was replaced with phenol-red free 1640-RPMI media containing 10% charcoal-stripped FBS and penicillin/streptomycin treated with constant ratios of either Compound I or fulvestrant as single agents, or a combination of both drugs at four different concentrations (2X IC50, 1X IC50, 0.5X IC50, 0.25X IC50), and incubated at 37 °C, 5% CO₂ for 7 days. The cells were retreated as described above on the 3rd or 4th day. Triplicate wells were used for each concentration and wells containing only media with 0.1% DMSO were used as a control. To measure cell viability, 100 uL of a 1:100 dilution of GF-AFC substrate into the Assay Buffer (CellTiter Fluor Cell Viability Assay (Promega)) were added to each well and incubated at 37 °C, 5% CO₂ for an additional 30-90 minutes. Fluorescence at 380-400 nm Excitation/505 nm Emission was read in a fluorometer and the percentage of cell titer relative to DMSO-treated cells was calculated after correcting for background by subtracting the blank well's signal. IC50 values for single agents were calculated using the GraphPad Prism software. Quantification of synergy was done by calculating combination indices (CI) using the CalcuSyn software (Biosoft) based on the Chou-Talalay algorithm (Chou and Talalay, 1984), and averaging the Cl values for the effective doses (ED) 50, 75, and 90. As shown in FIG. 6, addition of Compound I to fulvestrant resulted in improved inhibition of cell viability compared to either single agent with an average Cl value of 0.51.

## Claims

1. A BET bromodomain inhibitor selected from 1-benzyl-6-(3,5-dimethylisoxazol-4-yl)-N-methyl-1H-imidazo[4,5-b]pyridin-2-amine (Compound I) and pharmaceutically acceptable salts/co-crystals thereof, for use in a method of treating estrogen receptor positive (ER+) breast cancer in a patient, the method comprising administering said BET bromodomain inhibitor in combination with a second therapeutic agent, wherein the second therapeutic agent is fulvestrant or the second therapeutic agent is abemaciclib.

2. The inhibitor for use according to claim 1, wherein the patient previously has been treated with a breast cancer therapy.

3. The inhibitor for use according to claim 1, wherein the patient previously has been treated with a CDK4/6 inhibitor.

4. The inhibitor for use according to claim 1, wherein the patient previously has been treated with chemotherapy.

5. The inhibitor for use according to any one of claims 1-2, wherein the patient previously has been treated with immunotherapy.

6. The inhibitor for use according to any one of claims 1-5, wherein the patient is a human.

7. The inhibitor for use according to claim 1, wherein the BET bromodomain inhibitor is dosed with a second therapeutic agent without resulting in thrombocytopenia as a dose-limiting toxicity, wherein the second therapeutic agent is fulvestrant or abemaciclib.

## Patentansprüche

1. BET-Bromodomain-Inhibitor, ausgewählt aus 1-Benzyl-6-(3,5-dimethylisoxazol-4-yl)-N-methyl-1H-imidazo[4,5-b]pyridin-2-amin (Verbindung I)) und pharmazeutisch unbedenklichen Salzen/Kokristallen davon, zur Verwendung in einem Verfahren zur Behandlung von östrogenrezeptorpositivem (ER+) Brustkrebs bei einem Patienten, wobei das Verfahren ein Verabreichen des besagten BET-Bromodomain-Inhibitors in Kombination mit einem zweiten Therapeutikum umfasst, wobei das zweite Therapeutikum Fulvestrant ist oder das zweite Therapeutikum Abemaciclib ist.

2. Inhibitor zur Verwendung nach Anspruch 1, wobei der Patient zuvor mit einer Brustkrebstherapie behandelt wurde.

3. Inhibitor zur Verwendung nach Anspruch 1, wobei der Patient zuvor mit einem CDK4/6-Inhibitor behandelt wurde.

4. Inhibitor zur Verwendung nach Anspruch 1, wobei der Patient zuvor mit Chemotherapie behandelt wurde.

5. Inhibitor zur Verwendung nach einem der Ansprüche 1-2, wobei der Patient zuvor mit Immuntherapie behandelt wurde.

6. Inhibitor zur Verwendung nach einem der Ansprüche 1-5, wobei der Patient ein Mensch ist.

7. Inhibitor zur Verwendung nach Anspruch 1, wobei der BET-Bromodomain-Inhibitor mit einem zweiten Therapeutikum dosiert wird, ohne zu einer Thrombozytopenie als eine dosiseinschränkende Toxizität zu führen, wobei das zweite Therapeutikum Fulvestrant oder Abemaciclib ist.

## Revendications

1. Inhibiteur de bromodomaine de BET sélectionné parmi la 1-benzyl-6-(3,5-diméthylisoxazol-4-yl)-N-méthyl-1H-imidazo[4,5-b]pyridin-2-amine (Composé I) et des sels/co-cristaux pharmaceutiquement acceptables de celle-ci, destiné à une utilisation dans une méthode de traitement d'un cancer du sein positif pour les récepteurs aux oestrogènes (ER+) chez une patiente, la méthode comprenant l'administration dudit inhibiteur de bromodomaine de BET en combinaison avec un deuxième agent thérapeutique, le deuxième agent thérapeutique étant le fulvestrant ou le deuxième agent thérapeutique étant l'abémaciclib.

2. Inhibiteur destiné à une utilisation selon la revendication 1, la patiente ayant été traitée auparavant par un traitement contre le cancer du sein.

3. Inhibiteur destiné à une utilisation selon la revendication 1, la patiente ayant été traitée auparavant avec un inhibiteur de CDK4/6.

4. Inhibiteur destiné à une utilisation selon la revendication 1, la patiente ayant été traitée auparavant par chimiothérapie.

5. Inhibiteur destiné à une utilisation selon l'une quelconque des revendications 1 et 2, la patiente ayant été traitée auparavant par immunothérapie.

6. Inhibiteur destiné à une utilisation selon l'une quelconque des revendications 1 à 5, la patiente étant un humain.

7. Inhibiteur destiné à une utilisation selon la revendication 1, l'inhibiteur de bromodomaine de BET étant administré avec un deuxième agent thérapeutique sans entraîner de thrombocytopénie comme toxicité dose-limitante, le deuxième agent thérapeutique étant le fulvestrant ou l'abémaciclib.
